# EUROPÄISCHE PATENTSCHRIFT

(11) **EP 0 635 490 B1**
(45) Veröffentlichungstag und Bekanntmachung des Hinweises auf die Patenterteilung: **11.12.1996**
(21) Anmeldenummer: 94110725.2
(22) Anmeldetag: 11.07.1994
(51) Int. Cl.: C07D 209/86

(54) **Verfahren zur Herstellung von N-Ethylcarbazol**
Process for the preparation of N-ethylcarbazole
Procédé pour la préparation de N-éthylcarbazole

(30) Priorität: 23.07.1993 DE 4324707
(43) Veröffentlichungstag der Anmeldung: 25.01.1995
(73) Patentinhaber: HOECHST AKTIENGESELLSCHAFT, 65926 Frankfurt am Main (DE)
(72) Erfinder: Becherer, Johannes, Dr., D-63477 Maintal (DE); Koch, Peter, Dr., D-63179 Obertshausen (DE)

(56) Entgegenhaltungen:
- DE-A- 2 132 961
- SYNTHESIS, Nr.5, 1986, STUTTGART DE Seiten 382 - 383 M. LISSEL ET AL. 'Dimethylcarbonat als Methylierungsmittel unter phasentransfer-katalytischen Bedingungen'
- LIEBIGS ANNALEN DER CHEMIE, 1987, WEINHEIM DE Seiten 77 - 79 M. LISSEL 'N-Methylierung von Imidazol und Derivaten'

## Beschreibung

Die vorliegende Erfindung betrifft Verfahren zur Herstellung von N-Ethylcarbazol der Formel I aus einem Carbazol der allgemeinen Formel II, 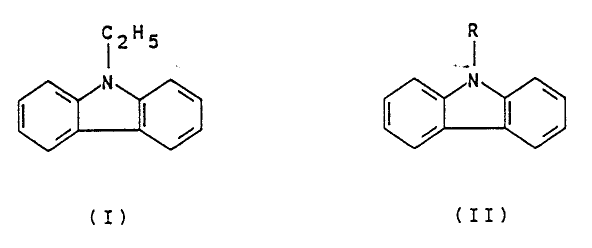
in der R Wasserstoff oder Ethoxycarbonyl bedeutet, durch Umsetzung mit Diethylcarbonat.

N-Ethylcarbazol ist ein wichtiges Zwischenprodukt zur Herstellung wertvoller Farbstoffe (vgl. Ullmanns Encyklopädie der technischen Chemie, 3. Auflage, Band 5, S. 80, 4. Auflage, Band 9, S. 120). Es wird technisch hergestellt durch Umsetzung von Carbazol mit Kaliumhydroxid oder Kaliumcarbonat zum Kaliumsalz des Carbazols, welches anschließend mit einem Ethylhalogenid (s. z.B. BIOS Final Report 986, S. 197) oder mit Diethylsulfat (s. z.B. DE-B-2132961) ethyliert wird. Andere Verfahren zur Herstellung von N-Ethylcarbazol aus Carbazol setzen beispielsweise Benzolsulfonsäureethylester (Chemical Abstracts, Band 82, Abstr. Nr. 45003 (1975)), Diethyl-N-(o-tolyl)phosphoramidat (Journal of Heterocyclic Chemistry, Band 18, S. 315 (1981)) oder 1,1-Diethoxyethyliumtetrafluoroborat (Liebigs Annalen der Chemie, 1987, S. 509) ein, sie haben aber keine technische Bedeutung.

Die bekannten technischen Herstellungsverfahren besitzen alle den Nachteil, daß große Mengen an anorganischen Salzen anfallen, die aus den Prozeßabwässern arbeits- und energieaufwendig durch Abwassereindampfung entfernt und dann deponiert werden müssen oder die mit den Abwässern über die Kläranlagen letztendlich in die Flüsse gelangen. Wird zum Beispiel nach dem im BIOS Final Report 986, S.197 beschriebenen Verfahren gearbeitet, so fällt ein Abwasser an, das pro Tonne produziertes N-Ethylcarbazol ca. 220 kg Kaliumchlorid enthält. Nach dem in der DE-B-2132961 beschriebenen Verfahren finden sich pro Tonne N-Ethylcarbazol sogar ca. 490 kg Kaliumsulfat in den Abwässern wieder. Die Verfahren, bei denen Ethylhalogenide als Ethylierungsmittel benutzt werden, haben noch den weiteren Nachteil, daß zur Vermeidung der Emission organischer Halogenverbindungen eine sehr aufwendige Abluftreinigung durchgeführt werden muß, da eine einfache Abluftverbrennung wegen des Halogengehalts nicht möglich ist. Auch läßt sich bei diesen Verfahren bei wäßriger Aufarbeitung des Reaktionsgemisches ein Gehalt an organischen Halogenverbindungen (AOX) im Abwasser nicht vermeiden. Darüberhinaus erfordern sowohl die Verwendung von Ethylhalogeniden als auch die von Diethylsulfat wegen der toxischen und cancerogenen Eigenschaften besondere Maßnahmen beim Umgang mit diesen Substanzen. Sowohl aus ökologischen als auch aus arbeitshygienischen Gründen wäre deshalb ein verbessertes Verfahren zur Herstellung von N-Ethylcarbazol äußerst wünschenswert.

Anstelle von Alkylhalogeniden oder Dialkylsulfaten können in Alkylierungen von Aminen teilweise Dialkylcarbonate eingesetzt werden, so ist beispielsweise die Verwendung von Dimethylcarbonat in Gegenwart von Phasentransferkatalysatoren wie Kronenethern anstelle von Dimethylsulfat bei der Methylierung von Imidazol in Liebigs Annalen der Chemie 1987, S.77, beschrieben. In dieser Veröffentlichung wird auf den ausgeprägten Reaktivitätsunterschied zwischen Dimethylcarbonat und Diethylcarbonat hingewiesen, mit Diethylcarbonat wurde nämlich in keinem Fall ein definiertes Produkt erhalten. Auf das unterschiedliche Verhalten von Dimethyl- und Diethylcarbonat und die mit letzterem erzielten schlechteren Ergebnisse wird beispielsweise auch in Synthesis 1986, S. 382, eingegangen. Ethylierungen am Stickstoffatom von Amidgruppen mittels Diethylcarbonat sind möglich, so ist in der europäischen Patentanmeldung EP-A-410214 die Umsetzung von Urethanen mit Diethylcarbonat in Gegenwart von mindestens äquivalenten Mengen von Alkali- oder Erdalkalicarbonaten und zusätzlich von Phasentransferkatalysatoren erwähnt, wegen der höheren Acidität der Amide ist dieses Reaktionsverhalten jedoch nicht mit dem von Aminen zu vergleichen.

Mit Aminen setzt sich Diethylcarbonat im allgemeinen zu Carbamidsäureestern um (Houben-Weyl, Methoden der organischen Chemie, Band E 4, S. 159; Ullmanns Encyklopädie der technischen Chemie, 4. Auflage, Band 14, S. 591; s. auch DE-B-2160111), eine Ethylierung wird dabei nur als Nebenreaktion beobachtet (US-Patent 4550188).

Nur in wenigen Fällen treten Ethylierungen bei Umsetzungen von aromatischen Aminen mit Diethylcarbonat als Hauptreaktion auf. So ist in der DE-A-2618033 neben Umsetzungen verschiedener Anilinderivate mit Dimethylcarbonat auch die Reaktion von p-Phenylendiamin und p-Toluidin, zwei durch elektronenschiebende Substituenten am Ring aktivierten Monoarylaminen, mit Diethylcarbonat beschrieben, bei der Gemische von am Stickstoff mono- und bisethylierten Produkten entstehen. Die Gasphasenreaktion von Anilin, einem relativ leichtflüchtigen aromatischen Amin, mit Diethylcarbonat in Gegenwart eines Polyethylenglykol und Kaliumcarbonat enthaltenden Katalysators liefert ein Gemisch aus 56,5 % N-Ethylanilin, 19,7 % N-Ethoxycarbonyl-N-ethylanilin und 24,4 % Anilin (Journal of Organic Chemistry, Band 52, S. 1300 (1987)), also einen hohen Anteil an nicht umgesetztem Ausgangsmaterial. Auf schwerflüchtige Amine ist diese Methode nicht übertragbar. Die Alkylierung aromatischer Amine mittels Dialkylcarbonaten in Gegenwart von organischen Iodiden als Katalysatoren ist in der deutschen Patentschrift DE-C-3007196 erwähnt. Eine technische Durchführung dieses Verfahrens würde wegen des Zusatzes der organischen Iodide aber wiederum eine aufwendige Abluftreinigung erforderlich machen und bei wäßriger Aufarbeitung zu einem Gehalt an organischen Halogenverbindungen im Abwasser führen. Zudem werden nur Umsetzungen mit Dimethylcarbonat offenbart, aus denen Gemische aus N-Methyl- und N,N-Dimethylanilinen erhalten werden. Es werden keine Hinweise gegeben, daß Diethylcarbonat ähnliche Ergebnisse liefert wie Dimethylcarbonat, das - wie bereits oben gesagt - bekanntermaßen wesentlich besser alkylierend wirkt. Das stark unterschiedliche Alkylierungsvermögen von Methyl- und Ethylgruppen in Kohlensäureestern geht auch aus der EP-B-104601 hervor. Dort wird für die N-Methylierung von Bis(2,4,6-tribromphenyl)amin zum gewünschten N,N-Bis-(2,4,6-tribromphenyl)methylamin neben der Verwendung von Dimethylcarbonat auch die Verwendung von gemischten Kohlensäureestern mit einer Methyl- und einer höheren Alkylgruppe, beispielsweise und bevorzugt einer Ethylgruppe, erwähnt. Von einer neben der Methylierung ablaufenden Alkylierung, insbesondere einer Ethylierung, die auf vergleichbares Reaktionsverhalten der Methyl- und Ethylgruppe in diesem Reaktionstyp der N-Alkylierung eines Diarylamins hindeuten würde, ist nicht die Rede.

Aber auch mit Dimethylcarbonat erfolgt zudem keine vollständige Umsetzung, so daß eine Trennung von Edukt und Produkt notwendig wird.

In der DE-C-1195756 schließlich ist die Umsetzung von aromatisch-heterocyclischen sekundären Aminen, beispielsweise Carbazol, mit Bis(dialkylaminoalkyl)carbonaten beschrieben. Die Besonderheiten dieser Umsetzung werden ausdrücklich erwähnt, insbesondere wird herausgestellt, daß diese spezielle Alkylierungsmethode nicht allgemein gültig für Ester der Kohlensäure ist, sondern spezifisch für die Kohlensäureester solcher aliphatischen Alkohole, die in der 2- oder der 3-Stellung eine tertiäre Aminogruppe tragen. Diese Aminogruppe dürfte intermediär am Alkoholkohlenstoffatom angreifen und durch diese Reaktionsbeteiligung maßgeblich für das beobachtete Verhalten sein. Entsprechend wird auch hier mit dem gemischten Kohlensäureester aus 3-Dimethylaminopropanol und Ethanol keine Ethylierung gefunden.

Angesichts des erläuterten Standes der Technik zur Ethylierung von Aminen mit Diethylcarbonat muß es als außerordentlich überraschend angesehen werden, daß es nun gelang, die Aufgabe, ein einfaches, die oben erwähnten ökologischen und arbeitshygienischen Nachteile der bekannten Verfahren vermeidendes neues technisches Herstellungsverfahren für N-Ethylcarbazol bereitzustellen, dadurch zu lösen, daß Carbazol oder auch N-Ethoxycarbonylcarbazol mit Diethylcarbonat umgesetzt wird. Gegenstand der vorliegenden Erfindung ist somit ein Verfahren zur Herstellung von N-Ethylcarbazol der Formel I, 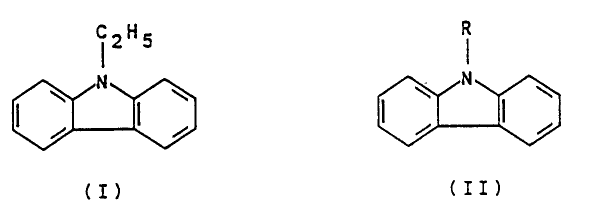
dadurch gekennzeichnet, daß ein Carbazol der allgemeinen Formel II, worin R Wasserstoff oder Ethoxycarbonyl bedeutet, mit Diethylcarbonat umgesetzt wird.

Als organische Nebenprodukte fallen bei dem erfindungsgemäßen Verfahren nur Kohlendioxid und Ethanol an. Die Abluft enthält neben Kohlendioxid Ethanol sowie Diethylcarbonat, welche daraus leicht mittels eines Wasserwäschers oder durch Abluftverbrennung entfernt werden können. Die ausgewaschenen organischen Verbindungen sind halogenfrei und sehr gut biologisch abbaubar, so daß der Wäscherinhalt über jede biologische Abwasserreinigungsanlage leicht entsorgt werden kann. Die Toxizität von Diethylcarbonat ist deutlich geringer als etwa die von Diethylsulfat, das cancerogene Potential geringer. Gegenüber Ethylchlorid hat Diethylcarbonat zudem den arbeitshygienischen Vorteil der geringeren Flüchtigkeit. Vorteilhaft an dem erfindungsgemäßen Verfahren ist weiterhin der glatte und vollständige Verlauf der Umsetzung, die in ausgezeichneter Ausbeute und hoher Raum-Zeit-Ausbeute ein sauberes Produkt liefert, das ohne zusätzliche Reinigungsoperation in die Folgestufen eingesetzt werden kann.

Das erfindungsgemäße Verfahren kann in der Form ausgeführt werden, daß das Carbazolderivat der allgemeinen Formel II mit Diethylcarbonat ohne weitere Zusätze zur Reaktion gebracht wird, beispielsweise durch Erwärmen auf die gewünschte Temperatur und mit der gewünschten Reaktionsführung. Die Reaktion kann aber auch in Gegenwart von Zusatzstoffen durchgeführt werden. So kann beispielsweise durch Zusatz einer mindestens äquivalenten Menge einer geeigneten Base das Carbazol der allgemeinen Formel II für den Fall, daß R für Wasserstoff steht, in sein Salz übergeführt sein (oder es kann auch für diesen Fall anstelle des Carbazols der allgemeinen Formel II ein vorab hergestelltes Salz zur Reaktion mit dem Diethylcarbonat gebracht werden). Erfindungsgemäß kann aber auch die Reaktion beispielsweise in Gegenwart einer geringeren als der äquivalenten Menge einer zugesetzten Base durchgeführt werden, also ohne vollständige Überführung des Carbazols der allgemeinen Formel II, in der R für Wasserstoff steht, in sein Salz. Günstig beeinflußt wird der Reaktionsablauf bereits durch katalytische Mengen basischer Verbindungen. In einer bevorzugten Ausführungsform wird somit die erfindungsgemäße Umsetzung der Substanzen der allgemeinen Formel II unter Basen-Katalyse durchgeführt. Eine Vielzahl anorganischer und organischer basischer Verbindungen ist als Katalysator allein oder im Gemisch mit anderen Basen geeignet.

Beispiele für geeignete Basen, die den weiten Bereich der einsetzbaren Verbindungen andeuten, sind Lithiumhydrid, Natriumhydrid, Kaliumhydrid, Magnesiumhydrid, Calciumhydrid, Methyllithium, Butyllithium, Phenyllithium, Organomagnesiumhalogenide, Dimsylnatrium, Tetraalkyl- und Aralkyltrialkylammoniumhydroxide wie Tetramethyl-, Tetraethyl-, Tetrabutyl-, Benzyltrimethyl- oder Benzyltriethylammoniumhydroxid, Salze von Carbonsäuren wie Natriumacetat, Kaliumacetat, Lithiumacetat, Natriumpropionat, Kaliumpropionat, Natriumbenzoat, Kaliumbenzoat, Kaliumphthalat, organische Stickstoffverbindungen, z.B. Imidazole wie Alkylimidazole, beispielsweise Methylimidazol, oder Diazabicyclononen oder Diazabicycloundecen, Lanthanhydroxid, Lanthanoxid, Lanthancarbonat, Lithium-, Natrium- und Kaliumborate, Lithium-, Natrium- und Kaliumsilicate, basische Ionenaustauscher, unter Verwendung von Vinylpyridinen hergestellte, Pyridylreste enthaltende Polymere.

Besonders bevorzugt sind als Katalysator tertiäre Amine, Pyridinderivate, Alkalimetallsalze von Aminen, Alkali- oder Erdalkalimetallsalze des Carbazols, Alkalimetallalkoholate, Erdalkalimetallalkoholate oder Hydroxide, Oxide, Carbonate oder Phosphate der Alkalimetalle oder Erdalkalimetalle oder Gemische dieser Substanzen.

Als Katalysator verwendete tertiäre Amine können verzweigte und unverzweigte aliphatische, cycloaliphatische, araliphatische und aromatische Reste enthalten, beispielsweise Methyl-, Ethyl-, n-Propyl-, i-Propyl-, n-Butyl-, i-Butyl-, Hexyl-, Octyl-, Decyl-, Dodecyl-, Hexadecyl-, Octadecyl-, Cyclopentyl-, Cyclohexyl-, Benzyl-, Phenethyl-, Phenyl-, Naphthyl-, Biphenylreste, in denen auch Kohlenstoffatome durch Stickstoff- oder Sauerstoffatome ersetzt sein können und die auch Substituenten wie Alkylgruppen oder Alkoxygruppen oder weitere tertiäre Aminogruppen enthalten können. Das Stickstoffatom des tertiären Amins kann auch Bestandteil von Ringen, beispielsweise von Fünf- oder Sechsringen oder von bi- oder tricyclischen Systemen, sein. Beispiele für geeignete tertiäre Amine sind Trialkylamine wie Triethylamin, Tributylamin, Trioctylamin, Tridecylamin, Ethyldiisopropylamin oder Decyldimethylamin, Aralkylamine wie Benzyldimethylamin oder Benzyldiethylamin, Aryldialkylamine wie N,N-Dimethylanilin, N,N-Diethylanilin oder N,N-Dibutylanilin, weiter substituierte Amine wie N,N,N',N'-Tetramethylethylendiamin, N,N,N',N'-Tetramethylpropylendiamin, 1,8-Bis(dimethylamino)naphthalin oder Tris(2-((2-methoxy)ethoxy)ethyl)amin, oder cyclische Amine wie N-Methylpyrrolidin, N-Ethylpyrrolidin, N-Phenylpyrrolidin, N-Ethylpiperidin, 1,2,2,6,6-Pentamethylpiperidin, N-Ethylmorpholin oder 1,4-Diazabicyclo(2,2,2)octan.

Als Pyridinderivate können beispielsweise das Pyridin selbst oder substituierte Pyridine wie die Picoline oder Lutidine oder wie das 4-Dimethylaminopyridin oder anellierte Pyridine wie Chinolin, Chinaldin, Acridin, 9-Dimethylaminoacridin oder wie Phenanthroline, z.B. 1,10-Phenanthrolin, eingesetzt werden.

Beispiele für Alkalimetallsalze von Aminen, die als Katalysatoren eingesetzt werden können, sind Natriumamid, Kaliumamid, Lithiumamid, Lithium-, Natrium- und Kaliumdimethyl-, -diethyl-, -diisopropyl- und -bistrimethylsilylamid, Kalium(3-aminopropyl)amid oder Lithiumcyclohexylamid, Beispiele für Alkali- oder Erdalkalimetallsalze des Carbazols, die als basische Katalysatoren eingesetzt werden können, sind das Lithiumsalz, das Natriumsalz, das Kaliumsalz, das Cesiumsalz, das Magnesiumsalz, das Calciumsalz oder das Bariumsalz des Carbazols.

Werden Alkalimetallalkoholate oder Erdalkalimetallalkoholate als Katalysatoren eingesetzt, so können sie als Metall beispielsweise Lithium, Natrium, Kalium, Cesium, Magnesium, Calcium oder Barium enthalten und von primären, sekundären oder tertiären Alkoholen abgeleitet sein, beispielsweise von Methanol, Ethanol, n-Propanol, i-Propanol, n-Butanol, i-Butanol, tert-Butanol, Amylalkoholen, Benzylalkohol, Cyclopentanol, Cyclohexanol oder auch mehrwertigen Alkoholen wie Ethylenglykol, Propylenglykol oder auch Polyethylen- oder Polypropylenglykolen. Geeignete Alkoholate sind also zum Beispiel Lithiummethylat, Natriummethylat, Kaliummethylat, Magnesiummethylat, Calciummethylat, Bariummethylat, Lithiumethylat, Natriumethylat, Kaliumethylat, Cesiumethylat, Magnesiumethylat, Calciumethylat, Bariumethylat, Lithiumisopropylat, Natriumisopropylat, Kaliumisopropylat, Lithium-tert-butylat, Natrium-tert-butylat oder Kalium-tert-butylat.

Beispiele für Hydroxide, Oxide, Carbonate und Phosphate der Alkalimetalle oder Erdalkalimetalle, die als Katalysatoren Verwendung finden können, sind Lithiumhydroxid, Lithiumoxid, Lithiumcarbonat, Lithiumphosphat, Natriumhydroxid, Natriumoxid, Natriumcarbonat, Natriumhydrogencarbonat, Natriumphosphat, Kaliumhydroxid, Kaliumoxid, Kaliumcarbonat, Kaliumhydrogencarbonat, Kaliumphosphat, Cesiumhydroxid, Cesiumcarbonat, Magnesiumhydroxid, Magnesiumoxid, Magnesiumcarbonat, Magnesiumhydroxidcarbonat, Magnesiumphosphat, Calciumhydroxid, Calciumoxid, Calciumcarbonat, Calciumphosphat, Bariumhydroxid, Bariumoxid, Bariumcarbonat, Bariumphosphat, wobei unter den Phosphaten basisch reagierende Salze der Monophosphorsäure, der Diphosphorsäure und von Polyphosphorsäuren zu verstehen sind, in denen Wasserstoffatome nur teilweise oder vollständig durch die Alkali- oder Erdalkalimetalle ersetzt sind. Die genannten Verbindungen können in wasserfreier Form, wasserhaltig, z.B. in Form ihrer Hydrate, oder auch als Lösung eingesetzt werden.

Die Menge des zugesetzten Katalysators richtet sich nach seiner katalytischen Aktivität und nach den gewählten weiteren Reaktionsparametern und kann in weiten Grenzen variiert werden. Größere Katalysatormengen schaden der Reaktion nicht. Wie bereits erwähnt, können Basen auch in äquivalenten Mengen zugesetzt werden, es kann aber auch ein Überschuß angewandt werden. So ist auch die gleichzeitige Verwendung katalytisch wirkender Basen als Lösungsmittel möglich. Bevorzugt wird der Katalysator in einer Menge von 0,05 bis 20 Molprozent, bezogen auf das eingesetzte Carbazol der allgemeinen Formel II, eingesetzt. Besonders bevorzugt ist eine Katalysatormenge von 0,1 bis 10 Molprozent. Diese Mengen führen bei Verwendung anorganischer Basen und wenn kein Wiedereinsatz des Katalysators erfolgt, in ökologisch vorteilhafter Weise nur noch zu einem geringen Anfall anorganischer Salze als Nebenprodukt.

Die Temperatur, bei der das Carbazolderivat der allgemeinen Formel II mit Diethylcarbonat umgesetzt wird, hängt vom Katalysatorzusatz und vom Reaktionsmedium ab. Bei Zusatz geeigneter Basen oder Basengemische kann die Umsetzung bereits bei Raumtemperatur oder unter Erwärmen bis auf z.B. 130°C ablaufen. Bevorzugt wird die Umsetzung zwischen 130°C und 320°C durchgeführt. Werden beispielsweise Pyridinderivate als Katalysatoren eingesetzt, kann bei niedrigeren Temperaturen gearbeitet werden. Besonders bevorzugt wird die erfindungsgemäße Umsetzung zwischen 180 und 300°C, darüber hinaus bevorzugt zwischen 220 und 280°C durchgeführt.

Die Reaktion kann unter einem Überdruck durchgeführt werden, wobei mittels eines Druckhalteventils ein bestimmter Überdruck kontrolliert eingehalten werden kann, z.B. ein Überdruck von maximal 3 bar oder maximal 6 bar, oder wobei unter dem sich einstellenden Druck gearbeitet werden kann, z.B., insbesondere bei Verwendung niedriger siedender Lösungsmittel, auch bei hohem Druck in einem Autoklaven. Bevorzugt wird die Reaktion des Carbazolderivats der allgemeinen Formel II mit Diethylcarbonat drucklos oder unter einem Überdruck von maximal 3 bar durchgeführt.

Die Reaktion kann in einem Lösungsmittel oder ohne Lösungsmittel durchgeführt werden. Wird kein Lösungsmittel verwandt, so wird das Carbazol der allgemeinen Formel II mit der für die Umwandlung in das Ethylcarbazol der Formel I einzusetzenden Menge Diethylcarbonat und gegebenenfalls unter Zusatz von basischen Katalysatoren bei der gewünschten Temperatur zur Reaktion gebracht. Wird eine größere als die für die Umwandlung erforderliche Menge Diethylcarbonat zugesetzt, so fungiert dieser Überschuß auch als Lösungsmittel. Das überschüssige Diethylcarbonat kann nach beendeter Umsetzung zurückgewonnen und wiedereingesetzt werden. Als Lösungsmittel können ansonsten alle Verbindungen eingesetzt werden, die unter den Reaktionsbedingungen bei der gewünschten Temperatur und gegen den gegebenenfalls zugesetzten basischen Katalysator stabil sind, beispielsweise aliphatische und aromatische Kohlenwasserstoffe wie z.B. Benzinfraktionen, Toluol, Xylol, auch Chlorkohlenwasserstoffe wie Chlorbenzol oder o-Dichlorbenzol, Ether wie z.B. Dibutylether, Ethylenglykolether wie Diethylenglykoldimethylether oder Triethylenglykoldimethylether, Dioxan, Azaaromaten wie Pyridin, Picoline, Lutidine, Chinolin, wobei diese Substanzen zugleich als basische Katalysatoren wirken, Amide wie Dimethylformamid oder N-Methylpyrrolidon, oder beispielsweise Dimethylsulfoxid oder Sulfolan. Als Lösungsmittel kann auch in vorteilhafter Weise geschmolzenes N-Ethylcarbazol verwandt werden, das nach beendeter Umsetzung zusammen mit dem neu gebildeten N-Ethylcarbazol wieder isoliert werden kann. Das Lösungsmittel kann auch ein Gemisch aus zwei oder mehr Komponenten sein.

Das erfindungsgemäße Verfahren kann in der Weise durchgeführt werden, daß man das Carbazol der allgemeinen Formel II mit dem Diethylcarbonat und gegebenenfalls einem basischen Katalysator und gegebenenfalls einem Lösungsmittel bei der gewünschten Temperatur hält, bis der Gehalt an Carbazol der allgemeinen Formel II, bezogen auf N-Ethylcarbazol, unter einem angestrebten Grenzwert, z.B. unter 1 % oder unter 0,1 %, liegt. Während der Reaktion wird Kohlendioxid freigesetzt. Das während der Reaktion freiwerdende Ethanol kann aus dem Reaktionsgemisch herausdestilliert werden. Die benötigte Reaktionstemperatur und die Reaktionsdauer sind abhängig von Art und Menge des Katalysators. Liegt die Reaktionstemperatur oberhalb des Siedepunktes des Lösungsmittels bzw. Reaktionsgemisches, muß unter Druck gearbeitet werden. Wird oberhalb des Siedepunktes des Diethylcarbonats gearbeitet, so wird vorteilhaft über eine Trennkolonne oder einen Partialkondensator, dessen Temperatur so gewählt ist, daß siedendes Diethylcarbonat in den Ansatz zurückfließt, die Dämpfe des entstandenen Ethanols jedoch den Kondensator passieren können, das Ethanol abdestilliert. Die Ethanoldämpfe und gegebenenfalls mitgerissenes Diethylcarbonat werden in einem nachgeschalteten Kühler kondensiert, wobei dieses Destillat gegebenenfalls noch destillativ in wiedereinsetzbares Diethylcarbonat und anderweitig verwertbares Ethanol zerlegt werden kann.

Bei einer bevorzugten Ausführungsform des erfindungsgemäßen Verfahrens wird nur ein Teil der Diethylcarbonatmenge, z.B. 5 - 50%, mit dem Carbazol der allgemeinen Formel II, gegebenenfalls einem Katalysator und gegebenenfalls einem Lösungsmittel vorgelegt und die Restmenge erst bei der Reaktionstemperatur nach und nach in dem Maße, in dem es verbraucht wird, zudosiert, oder es werden nur das Carbazol der allgemeinen Formel II, gegebenenfalls ein Katalysator und gegebenenfalls ein Lösungsmittel vorgelegt und das gesamte Diethylcarbonat wird bei der Reaktionstemperatur nach und nach zudosiert, wobei, wenn oberhalb der Siedetemperatur des Diethylcarbonats gearbeitet wird, der Rücklauf aus einem bei geeigneter Temperatur betriebenen Rückflußkühler als Maß für den Verbrauch dienen kann.

Nach beendeter Umsetzung kann die Aufarbeitung in der Weise erfolgen, daß gegebenenfalls feste Katalysatoren bei geeigneter Temperatur abfiltriert werden und/oder gegebenenfalls flüchtige Bestandteile wie Lösungsmittel, flüchtige Katalysatoren, Diethylcarbonat und Ethanol destillativ bei geeignetem Druck, z.B. bei Normaldruck oder im Vakuum, abgetrennt werden. Das verbliebene Produkt kann im allgemeinen direkt zur Weiterverarbeitung verwendet werden. Bei Bedarf kann es gegebenenfalls nochmals durch Filtration der Schmelze von geringen Mengen von Salzen befreit werden oder auch z.B. durch Extraktion, beispielsweise Extraktion von Salzen aus dem Produkt mit heißem Wasser, durch Kristallisation oder durch Destillation gereinigt werden. Das wiedergewonnene Diethylcarbonat und Lösungsmittel und der wiedergewonnene Katalysator können beim folgenden Ansatz wiedereingesetzt werden.

Bevorzugt wird das erfindungsgemäße Verfahren zur Herstellung von N-Ethylcarbazol dergestalt ausgeführt, daß Carbazol der Formel IIa 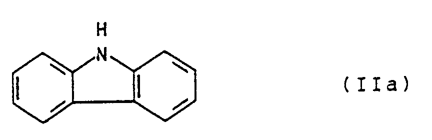
mit Diethylcarbonat umgesetzt wird, wobei als Katalysator Hydroxide, Oxide, Carbonate, Phosphate oder Ethylate der Alkalimetalle oder der Erdalkalimetalle oder die von diesen Metallen mit Carbazol gebildeten Salze oder Gemische dieser Verbindungen eingesetzt werden und wobei die Umsetzung in geschmolzenem N-Ethylcarbazol oder in Diethylcarbonat oder in einer Mischung hieraus durchgeführt wird. Die Verwendung der genannten Katalysatoren, für die Beispiele bereits angeführt wurden und die nach der Reaktion durch Filtrieren wiedergewonnen werden können, hat gegenüber der Verwendung anderer basischer organischer Verbindungen, welche ebenfalls als Katalysatoren dienen könnten, den besonderen Vorteil, daß im Reaktionsgemisch am Ende außer dem Produkt und den zwangsläufig anfallenden flüchtigen, destillativ leicht abtrennbaren Komponenten Ethanol und Diethylcarbonat sowie eventuellen, aus dem eingesetzten Carbazol stammenden, nicht störenden Verunreinigungen keine zusätzlichen organischen Bestandteile enthalten sind. Dies erspart aufwendigere Aufarbeitungs- und Reinigungsoperationen. Aus dem gleichen Grunde ist auch die Verwendung von N-Ethylcarbazol oder von Diethylcarbonat, das dann also im Überschuß eingesetzt wird, als Reaktionsmedium besonders vorteilhaft. Andere Lösungsmittel, in denen die Reaktion auch durchgeführt werden könnte, würden einen höheren Wiederaufarbeitungsaufwand erfordern. Bei der besonders bevorzugten Arbeitsweise, bei der N-Ethylcarbazol als Reaktionsmedium dient, kann nach Beendigung der Reaktion auch nur ein Teil, z.B. 30 - 70 %, bevorzugt 50 - 70 %, des Reaktionsgemisches aus dem Ethylierungsgefäß abgezogen und aufgearbeitet werden, während der Rest als Reaktionsmedium für den nächsten Ansatz im Ethylierungsgefäß zurückbleibt. Dabei kann durch Absitzen des Katalysators vor der Entnahme des zur Aufarbeitung vorgesehenen Teils des Reaktionsgemisches der Katalysator weitgehend im Reaktionsgefäß gehalten und dadurch mehrmals verwendet werden, was die Salzmenge im Abwasser noch weiter verringert.

Von den genannten Katalysatoren dieser bevorzugten Ausführungsform, bei der Carbazol der Formel IIa mit Diethylcarbonat umgesetzt wird, sind Kaliumhydroxid, Kaliumcarbonat und Kaliumethylat besonders bevorzugt, davon ganz besonders Kaliumhydroxid und Kaliumcarbonat, jeweils allein oder im Gemisch miteinander. Besonders bevorzugt durchgeführt wird die bevorzugte Ausführungsform im Temperaturbereich zwischen 130 und 320°C, ganz besonders bevorzugt zwischen 180 und 300°C und insbesondere zwischen 220 und 280°C. Vorteilhaft wird das Diethylcarbonat bei der bevorzugten Ausführungsform im Überschuß eingesetzt, auch wenn N-Ethylcarbazol als Reaktionsmedium verwendet wird, besonders bevorzugt in einer Menge von 1,1 bis 2,5 Mol, ganz besonders bevorzugt 1,1 bis 1,8 Mol, bezogen auf das eingesetzte Carbazol der Formel IIa. Der nicht verbrauchte Anteil kann nach Beendigung der Reaktion destillativ wiedergewonnen werden.

Bevorzugt ausgeführt wird das erfindungsgemäße Verfahren zur Herstellung von N-Ethylcarbazol zudem dergestalt, daß N-Ethoxycarbonylcarbazol der Formel IIb 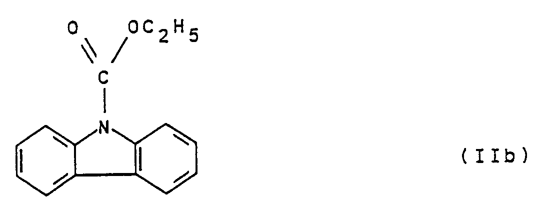
mit Diethylcarbonat umgesetzt wird, wobei das Diethylcarbonat in einer Menge von 0,1 bis 1 Mol, bevorzugt 0,3 bis 0,8 Mol, bezogen auf das eingesetzte Carbazol der Formel IIb, eingesetzt wird und wobei die Umsetzung ohne ein zusätzliches Lösungsmittel durchgeführt wird. Bei der besonders bevorzugten Arbeitsweise dieser Ausführungsform, bei der unter Basen-Katalyse gearbeitet wird, gelten für die Auswahl des Katalysators die bereits angestellten Überlegungen entsprechend. Auch hier erspart die bevorzugte Durchführung der Umsetzung in einer Schmelze des N-Ethoxycarbonylcarbazols bzw. in Diethylcarbonat und der Verzicht auf ein zusätzliches Lösungsmittel, in dem die Reaktion auch durchgeführt werden könnte, einen höheren Aufarbeitungsaufwand. Die Herstellung der Ausgangssubstanz dieser bevorzugten Ausführungsform, des N-Ethoxycarbonylcarbazols, ist in der US-Patentschrift 2089985 beschrieben.

Die nachfolgenden Beispiele dienen der Erläuterung der erfindungsgemäßen Verfahren.

### Beispiel 1:

In einem 250-ml-Mehrhalskolben mit einem Rückflußkühler, der mit Wasser von 90°C betrieben wird und auf den ein mit Eiswasser betriebener Liebig-Kühler aufgesetzt ist, werden 81 g N-Ethylcarbazol, technische 96,5%ige Ware, aufgeschmolzen. Bei einer Temperatur von 100 - 130°C werden 69,5 g Carbazol, technische 96%ige Ware, und 3 g Kaliummethylat unter gutem Rühren eingetragen. Die Suspension wird im Verlaufe von einer Stunde auf 230 - 240°C hochgeheizt, wobei bei einer Innentemperatur von 180 - 200°C mit dem Zutropfen von Diethylcarbonat begonnen wird. Die Innentemperatur wird 24 Stunden bei 230 - 240°C gehalten und es wird in dem Maße Diethylcarbonat zugetropft, daß dieses ständig am Rückfluß siedet. Insgesamt werden ca. 70 g gebraucht. Während der Reaktion entweicht Kohlendioxid und es destilliert Ethanol, verunreinigt mit etwas Diethylcarbonat und Methanol, ab. Sobald die Reaktion beendet ist, wird durch Anlegen von Vakuum das überschüssige Diethylcarbonat abdestilliert und die verbleibende Produktschmelze bei Temperaturen von 70 - 100°C mit Wasser ausgerührt. Nach dem Trocknen der organischen Phase durch Abdampfen der Restfeuchtigkeit erhält man 161 g N-Ethylcarbazol 96%ig, das entspricht unter Berücksichtigung der eingesetzten Menge einer Ausbeute von 98% der Theorie. Das Abwasser enthält nach Neutralisation mit Salzsäure 3,2 g Kaliumchlorid, das entspricht 42 kg Kaliumchlorid pro Tonne hergestelltes N-Ethylcarbazol.

### Beispiel 2:

Arbeitet man wie in Beispiel 1, befreit den Ansatz jedoch durch Filtration der Produktschmelze bei Temperaturen um 100°C vom Katalysator, so erhält man 158 g N-Ethylcarbazol, 96%ig, das entspricht unter Berücksichtigung der eingesetzten Menge einer Ausbeute von 95%. Nicht berücksichtigt ist dabei das Produkt, das dem Filterrückstand anhaftet.

### Beispiel 3:

Arbeitet man wie in Beispiel 1, setzt jedoch als Katalysator an Stelle von Kaliummethylat 4 g Kalium-tert-butylat zu, erhält man N-Ethylcarbazol in gleicher Ausbeute und Qualität.

### Beispiel 4:

Arbeitet man wie in Beispiel 1, setzt jedoch bei einer Reaktionstemperatur von 250 - 260°C als Katalysator an Stelle von Kaliummethylat 7 g Kaliumsalz des Carbazols zu, so erhält man nach 20 Stunden Reaktionszeit 162 g N-Ethylcarbazol mit einem Reingehalt von 96,5%.

### Beispiel 5:

Arbeitet man wie in Beispiel 1, setzt jedoch als Katalysator an Stelle von Kaliummethylat 2 g 4-Dimethylaminopyridin zu, erhitzt 24 Stunden auf 160 - 170°C und arbeitet durch Vakuumdestillation auf, so erhält man nach Abtrennen eines Vorlaufs, welcher aus Dimethylaminopyridin und N-Ethylcarbazol besteht und ggf. in einem Folgeansatz wieder eingesetzt werden kann, 155 g 96%iges N-Ethylcarbazol, das entspricht einer Ausbeute von 91%, wobei das im Vorlauf enthaltene Produkt nicht berücksichtigt ist.

### Beispiel 6:

In der in Beispiel 1 beschriebenen Apparatur werden in vorgelegte 50 ml o-Dichlorbenzol 35 g Carbazol 96%ig eingetragen und gelöst. Es werden 36 g Diethylcarbonat und 1 g 4-Dimethylaminopyridin zugegeben und alles zum Rückfluß erhitzt. Es stellt sich eine Innentemperatur von ca. 150°C ein, welche im Laufe der Zeit auf ca. 167 - 170°C steigt. Nach 24 Stunden wird 1 g Katalysator nachgesetzt. Nach ca. 48 Stunden am Rückfluß ist die Reaktion beendet und kann analog Beispiel 5 destillativ aufgearbeitet werden.

### Beispiel 7:

Arbeitet man wie in Beispiel 4, setzt jedoch an Stelle des Kaliumsalzes des Carbazols als Katalysator 5 g N,N-Dimethylanilin zu, so ist die Reaktion nach ca. 40 Stunden beendet.

### Beispiel 8:

Arbeitet man wie in Beispiel 1, jedoch völlig ohne Zusatz eines Katalysators und bei Temperaturen von 270 - 310°C, so ist die Reaktion nach ca. 40 Stunden beendet.

### Beispiel 9:

In einem 2-l-Mehrhalskolben mit einem Rückflußkühler, der mit Wasser von 90°C betrieben wird und auf den ein mit Eiswasser betriebener Liebig-Kühler aufgesetzt ist, werden 505 g N-Ethylcarbazol, technische 96,5%ige Ware, aufgeschmolzen. Bei einer Temperatur von 100 - 130°C werden 1044 g Carbazol, technische 96%ige Ware, und 30 g Kaliumhydroxid 90%ig unter gutem Rühren eingetragen. Die Suspension wird im Verlaufe von einer Stunde auf 230 - 240°C hochgeheizt, wobei bei einer Innentemperatur von 180 - 200°C mit dem Zutropfen von Diethylcarbonat begonnen wird. Die Innentemperatur wird 24 Stunden bei 230 - 240°C gehalten und es wird in dem Maße Diethylcarbonat zugetropft, daß dieses ständig am Rückfluß siedet. Insgesamt werden ca. 1000 g gebraucht. Während der Reaktion entweicht Kohlendioxid und es destilliert Ethanol mit etwas Diethylcarbonat ab. Sobald die Reaktion beendet ist, wird durch Anlegen von Vakuum das überschüssige Diethylcarbonat abdestilliert und die verbleibende Produktschmelze bei Temperaturen um 100^{o}C durch Filtration vom Katalysator befreit. Der Filterrückstand wird mit heißem Wasser behandelt, wobei die anorganischen Bestandteile Kaliumhydroxid und Kaliumcarbonat in Lösung gehen. Anhaftendes Produkt wird entweder heiß als organische Phase abgetrennt oder nach Erkalten durch Filtration isoliert. Man erhält insgesamt 1706 g N-Ethylcarbazol, 96,5%ig, davon 1630 g als filtrierte Schmelze, den Rest aus der wäßrigen Aufarbeitung des Filterrückstands, das ist unter Berücksichtigung der eingesetzten Menge eine Ausbeute von 99%. Als Verunreinigungen enthält das Produkt lediglich die Nebenkomponenten, die schon im technischen Carbazol enthalten waren.
Nach Neutralisation mit Salzsäure enthält das bei der Aufarbeitung des Filterrückstands angefallene Abwasser ca. 36 g Kaliumchlorid, das entspricht 31 kg pro Tonne hergestelltes N-Ethylcarbazol 100%ig.

### Beispiel 10:

Man arbeitet wie in Beispiel 9, an Stelle von 505 g N-Ethylcarbazol, 1044 g Carbazol und 30 g Kaliumhydroxid werden aber 500 g N-Ethylcarbazol, 800 g Carbazol und 2,5 g Kaliumhydroxid eingesetzt. Die Innentemperatur wird nur ca. 4 Stunden bei 230 - 240°C gehalten und dann auf 260 - 270°C erhöht. Es werden ca. 650 g Diethylcarbonat verbraucht. Die Reaktion ist ca. 16 Stunden nach Beginn der Diethylcarbonatzugabe beendet. Nach Aufarbeitung wie in Beispiel 9 erhält man 1420 g N-Ethylcarbazol, 96,5%ig, davon 1415 g als filtrierte Schmelze. Das neutralisierte Abwasser enthält ca. 3 g Kaliumchlorid, das entspricht ca. 3 - 4 kg pro Tonne hergestelltes N-Ethylcarbazol 100%ig.

### Beispiel 11:

Zunächst wurde ein Ansatz analog Beispiel 10, jedoch mit 10 g Kaliumhydroxid als Katalysator, durchgeführt. Nach Beendigung der Reaktion wurde der Rührer abgestellt und der Katalysator absitzen gelassen. Dann wurde das Reaktionsgemisch bis auf 500 g aus dem Kolben abgehebert und aufgearbeitet.
Das im Kolben verbliebene Rohgemisch einschließlich Katalysator wurde als Reaktionsmedium für den nächsten Ansatz benutzt, bei dem wiederum wie oben verfahren wurde. Insgesamt wurden auf diese Weise fünf Folgeansätze durchgeführt ohne weitere Katalysatorzugabe. Bis zu diesem Zeitpunkt konnte noch keine Verminderung der Katalysatoraktivität festgestellt werden.
Durchschnittlicher Salzanfall bis dahin 2 - 2,5 kg Kaliumchlorid pro Tonne N-Ethylcarbazol oder die entsprechende Menge eines Gemisches aus Kaliumhydroxid und Kaliumcarbonat, falls das Abwasser nicht mit Salzsäure neutralisiert wird. Durch weitere Folgeansätze bis zu einer feststellbaren Verminderung der Katalysatoraktivität kann der durchschnittliche Salzanfall pro Tonne Produkt weiter verringert werden.

### Beispiel 12:

Arbeitet man wie in Beispiel 10, setzt aber als Katalysator nur 0,3 g Kaliumhydroxid ein, so dauert die Reaktion bis zur vollständigen Umsetzung ca. 30 Stunden bei gleicher Ausbeute und Produktqualität. Der Salzanfall liegt unter 0,5 kg pro Tonne N-Ethylcarbazol.

### Beispiel 13:

Arbeitet man wie in Beispiel 9, jedoch bei einer Reaktionstemperatur von 260 - 270°C, und setzt an Stelle des Kaliumhydroxids als Katalysator 60 g Kaliumcarbonat ein, so erhält man nach einer Reaktionsdauer von 20 Stunden N-Ethylcarbazol in gleicher Ausbeute und Qualität.

### Beispiel 14:

Arbeitet man wie in Beispiel 4, verwendet jedoch als Katalysator 3 g Trikaliumphosphat, so ist die Reaktion nach ca. 50 Stunden beendet.

### Beispiel 15:

Arbeitet man wie in Beispiel 4, verwendet jedoch als Katalysator 12 g Bariumhydroxid-Octahydrat, so ist die Reaktion nach ca. 70 Stunden beendet.

### Beispiel 16:

Arbeitet man wie in Beispiel 4, verwendet jedoch als Katalysator 10 g Kaliumcarbonat, so erhält man nach einer Reaktionsdauer von ca. 20 Stunden N-Ethylcarbazol in gleicher Ausbeute mit einem Reingehalt von 96,5%.

### Beispiel 17:

Arbeitet man wie in Beispiel 1, setzt jedoch bei einer Reaktionstemperatur von 260 - 270°C an Stelle von Kaliummethylat als Katalysator 5 g Natriumethylat ein, so erhält man nach einer Reaktionsdauer von ca. 40 Stunden N-Ethylcarbazol in vergleichbarer Ausbeute mit einem Reingehalt von 95%.

### Beispiel 18:

In der in Beispiel 1 beschriebenen Apparatur werden 87 g Carbazol 96%ig mit 0,3 g Kaliumhydroxid aufgeschmolzen (Schmelzbereich um 245°C). In die Schmelze wird bei Temperaturen von 245 - 265°C Diethylcarbonat in dem Maße zugetropft, daß es ständig am Rückfluß kocht. In den ersten Stunden muß hochsublimiertes Carbazol gelegentlich abgeschmolzen werden. Nach ca. 20 Stunden ist die Umsetzung vollständig und liefert nach Aufarbeitung analog Beispiel 9 N-Ethylcarbazol mit einem Reingehalt von 96,5%.

### Beispiel 19:

In einem 2-l-Mehrhalskolben mit einem Rückflußkühler, der mit Wasser von 90^{o}C betrieben wird und auf den ein mit Eiswasser betriebener Liebig-Kühler aufgesetzt ist, werden 830 g Diethylcarbonat vorgelegt und 1044 g Carbazol 96%ig und 40 g Kaliumethylat eingetragen. Die Suspension wird erhitzt. Bei einer Innentemperatur von ca. 130°C beginnt der Ansatz am Rückfluß zu sieden. Im Verlaufe der Reaktion steigt die Innentemperatur an und erreicht nach ca. 4 Std. 230°C. Der Ansatz wird weitere 24 Std. bei 230 - 240°C gehalten, wobei weiteres Diethylcarbonat in dem Maße zugesetzt wird, daß es immer am Rückfluß kocht. Nach Beendigung der Reaktion wird wie in Beispiel 9 aufgearbeitet. Man erhält 1140 g filtrierte Produktschmelze und weitere 70 g Produkt aus der Aufarbeitung des Filterrückstands. Reingehalt 95%, Ausbeute 98% der Theorie.

### Beispiel 20:

In einem 100-ml-Kolben mit Rückflußkühler werden 47,8 g Carbazol-N-carbonsäureethylester aufgeschmolzen, mit 1 g Kaliumhydroxid und 5 g Diethylcarbonat versetzt und auf 230 - 240°C erhitzt, wobei weitere 10 g Diethylcarbonat in der Weise zugetropft werden, daß bei einer Innentemperatur von 230 - 240°C Diethylcarbonat ständig schwach am Rückfluß kocht. Es entweicht Kohlendioxid. Nach 24 Stunden ist die Reaktion beendet. Nach Abdestillieren des Diethylcarbonats im Vakuum und Entfernen des Katalysators durch Filtration der Schmelze erhält man 37 g N-Ethylcarbazol, frei von Carbazol und Carbazol-N-carbonsäureethylester.

### Beispiel 21:

Arbeitet man wie in Beispiel 20, setzt jedoch bei einer Reaktionstemperatur von 250 - 260°C an Stelle von Kaliumhydroxid als Katalysator 1 g Kaliummethylat ein, so erhält man nach einer Reaktionszeit von 5 Stunden und üblicher Aufarbeitung N-Ethylcarbazol mit einem Reingehalt von 97%.

## Patentansprüche

1. Verfahren zur Herstellung von N-Ethylcarbazol der Formel I, dadurch gekennzeichnet, daß ein Carbazol der allgemeinen Formel II, 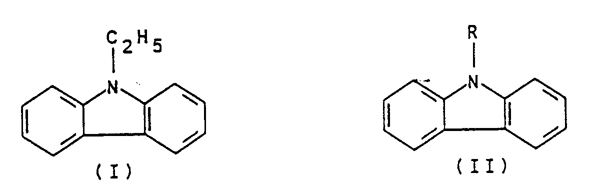 worin R Wasserstoff oder Ethoxycarbonyl bedeutet, mit Diethylcarbonat umgesetzt wird.

2. Verfahren gemäß Anspruch 1, dadurch gekennzeichnet, daß die Umsetzung unter Basen-Katalyse durchgeführt wird, wobei als Katalysator tertiäre Amine, Pyridinderivate, Alkalimetallsalze von Aminen, Alkali- oder Erdalkalimetallsalze des Carbazols, Alkalimetallalkoholate, Erdalkalimetallalkoholate oder Hydroxide, Oxide, Carbonate oder Phosphate der Alkalimetalle oder Erdalkalimetalle oder Gemische dieser Substanzen bevorzugt sind.

3. Verfahren gemäß Anspruch 2, dadurch gekennzeichnet, daß der Katalysator in einer Menge von 0,05 bis 20 Molprozent, bevorzugt 0,1 bis 10 Molprozent, bezogen auf das eingesetzte Carbazol der allgemeinen Formel II, eingesetzt wird.

4. Verfahren gemäß einem oder mehreren der Ansprüche 1 bis 3, dadurch gekennzeichnet, daß die Umsetzung zwischen 130 und 320°C, bevorzugt zwischen 180 und 300°C, besonders bevorzugt zwischen 220 und 280°C durchgeführt wird.

5. Verfahren gemäß einem oder mehreren der Ansprüche 1 bis 4, dadurch gekennzeichnet, daß das Carbazol der allgemeinen Formel II, gegebenenfalls ein Katalysator, gegebenenfalls ein Lösungsmittel und gegebenenfalls ein Teil des Diethylcarbonats vorgelegt werden und das Diethylcarbonat oder gegebenenfalls das restliche Diethylcarbonat während der Reaktion bei der Reaktionstemperatur zudosiert wird.

6. Verfahren gemäß einem oder mehreren der Ansprüche 1 bis 5, dadurch gekennzeichnet, daß Carbazol der Formel IIa 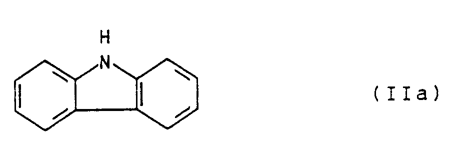 mit Diethylcarbonat umgesetzt wird, wobei als Katalysator Hydroxide, Oxide, Carbonate, Phosphate oder Ethylate der Alkalimetalle oder der Erdalkalimetalle oder die von diesen Metallen mit Carbazol gebildeten Salze oder Gemische dieser Verbindungen eingesetzt werden und wobei die Umsetzung in geschmolzenem N-Ethylcarbazol oder in Diethylcarbonat oder in einer Mischung hieraus durchgeführt wird.

7. Verfahren gemäß Anspruch 6, wobei als Katalysator Kaliumhydroxid, Kaliumcarbonat oder Kaliumethylat oder Gemische dieser Verbindungen eingesetzt werden.

8. Verfahren gemäß Anspruch 6 und/oder 7, dadurch gekennzeichnet, daß die Umsetzung zwischen 130 und 320°C durchgeführt wird.

9. Verfahren gemäß einem oder mehreren der Ansprüche 6 bis 8, dadurch gekennzeichnet, daß das Diethylcarbonat in einer Menge von 1,1 bis 2,5 Mol, bevorzugt 1,1 bis 1,8 Mol, bezogen auf das eingesetzte Carbazol der Formel IIa, eingesetzt wird.

10. Verfahren gemaß einem oder mehreren der Ansprüche 1 bis 5, dadurch gekennzeichnet, daß N-Ethoxycarbonylcarbazol der Formel IIb 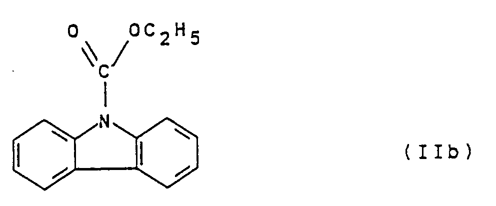 mit Diethylcarbonat umgesetzt wird, wobei das Diethylcarbonat in einer Menge von 0,1 bis 1,0 Mol, bevorzugt 0,3 bis 0,8 Mol, bezogen auf das eingesetzte Carbazol der Formel IIb, eingesetzt wird und wobei die Umsetzung ohne ein zusätzliches Lösungsmittel durchgeführt wird.

## Claims

1. A process for preparing N-ethylcarbazole of the formula I, which comprises reacting a carbazole of the formula II, 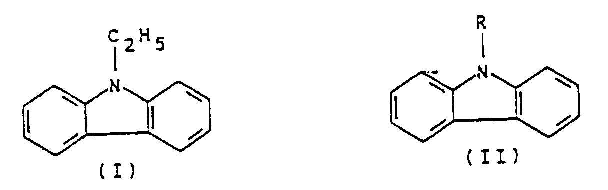 where R is hydrogen or ethoxycarbonyl, with diethyl carbonate.

2. The process as claimed in claim 1, wherein the reaction is carried out under base catalysis, preferred catalysts being tertiary amines, pyridine derivatives, alkali metal salts of amines, alkali metal or alkaline earth metal salts of carbazole, alkali metal alkoxides, alkaline earth metal alkoxides or hydroxides, oxides, carbonates or phosphates of the alkali metals or alkaline earth metals or mixtures of these substances.

3. The process as claimed in claim 2, wherein the catalyst is used in an amount of from 0.05 to 20 mol percent, preferably from 0.1 to 10 mol percent, based on the carbazole of the formula II used.

4. The process as claimed in one or more of claims 1 to 3, wherein the reaction is carried out between 130 and 320°C, preferably between 180 and 300°C, particularly preferably between 220 and 280°C.

5. The process as claimed in one or more of claims 1 to 4, wherein the carbazole of the formula II, if desired a catalyst, if desired a solvent and if desired a part of the diethyl carbonate are initially charged and the diethyl carbonate, or if applicable the remaining diethyl carbonate, is metered in at the reaction temperature during the reaction.

6. The process as claimed in one or more of claims 1 to 5, wherein carbazole of the formula IIa 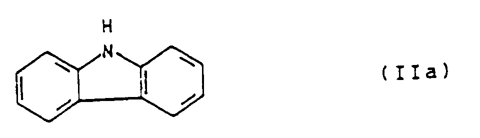 is reacted with diethyl carbonate, where catalysts used are hydroxides, oxides, carbonates, phosphates or ethoxides of the alkali metals or the alkaline earth metals or the carbazole salts formed from these metals or mixtures of these compounds and where the reaction is carried out in molten N-ethylcarbazole or in diethyl carbonate or in a mixture thereof.

7. The process as claimed in claim 6, wherein the catalyst used is potassium hydroxide, potassium carbonate or potassium ethoxide or a mixture of these compounds.

8. The process as claimed in claim 6 and/or 7, wherein the reaction is carried out between 130 and 320°C.

9. The process as claimed in one or more of claims 6 to 8, wherein the diethyl carbonate is used in an amount of from 1.1 to 2.5 mol, preferably 1.1 to 1.8 mol, based on the carbazole of the formula IIa used.

10. The process as claimed in one or more of claims 1 to 5, wherein N-ethoxycarbonylcarbazole of the formula IIb 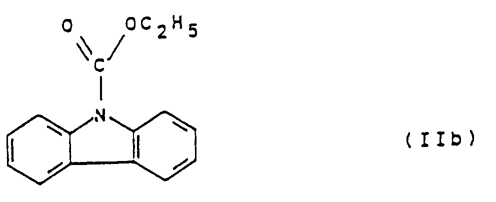 is reacted with diethyl carbonate, where the diethyl carbonate is used in an amount of from 0.1 to 1.0 mol, preferably from 0.3 to 0.8 mol, based on the carbazole of the formula IIb used, and where the reaction is carried out without an additional solvent.

## Revendications

1. Procédé pour la préparation de N-éthylcarbazole de formule I caractérisé en ce qu'on fait réagir un carbazole de formule générale II 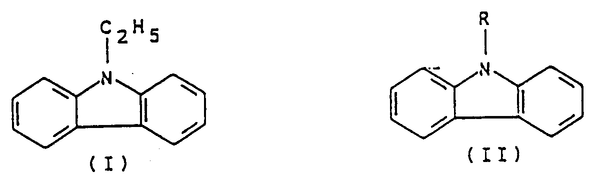 où R représente l'hydrogène ou éthoxycarbonyle, avec le carbonate de diéthyle.

2. Procédé selon la revendication 1, caractérisé en ce qu'on met en oeuvre la réaction sous catalyse en utilisant des bases, en préférant en tant que catalyseurs des amines tertiaires, des dérivés de la pyridine, des sels de métaux alcalins des amines, des sels de métaux alcalins ou alcalino-terreux du carbazole, des alcoolates de métaux alcalins, des alcoolates de métaux alcalino-terreux ou des hydroxydes, des oxydes, des carbonates ou des phosphates de métaux alcalins ou de métaux alcalino-terreux ou des mélanges de ces substances.

3. Procédé selon la révendication 2, caractérisé en ce qu'on utilise le catalyseur dans une quantité de 0,05 à 20 % en moles, de préférence de 0,1 à 10 % en moles, par rapport au carbazole de formule générale II utilisé.

4. Procédé selon une ou plusieurs des revendications 1 à 3, caractérisé en ce qu'on met en oeuvre la réaction à une température comprise entre 130 et 320°C, de préférence entre 180 et 300°C, de manière particulièrement préférée entre 220 et 280°C.

5. Procédé selon une ou plusieurs des revendications 1 à 4, caractérisé en ce qu'on introduit le carbazole de formule générale II, éventuellement un catalyseur, éventuellement un solvant et éventuellement une partie de carbonate de diéthyle et on ajoute progressivement le carbonate de diéthyle ou éventuellement le reste du carbonate de diéthyle au cours de la réaction à la température de réaction.

6. Procédé selon une ou plusieurs des revendications 1 à 5, caractérisé en ce qu'on fait réagir le carbazole de formule IIa 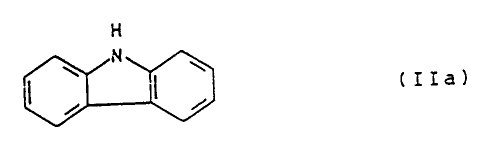 avec le carbonate de diéthyle, en utilisant en tant que catalyseur des hydroxydes, des oxydes, des carbonates, des phosphates ou des éthylates de métaux alcalins ou de métaux alcalino-terreux, ou des sels de ces métaux avec le carbazole ou des mélanges de ces composés, et en mettant en oeuvre la réaction dans du N-éthylcarbazole fondu ou du carbonate de diéthyle ou dans un mélange de ceux-ci.

7. Procédé selon la revendication 6, en utilisant en tant que catalyseur l'hydroxyde de potassium, le carbonate de potassium ou le méthylate de potassium ou des mélanges de ces composés.

8. Procédé selon la revendication 6 et/ou 7, caractérisé en ce qu'on met en oeuvre la réaction à une température comprise entre 130 et 320°C.

9. Procédé selon une ou plusieurs des revendications 6 à 8, caractérisé en ce qu'on utilise le carbonate de diéthyle dans une quantité de 1,1 à 2,5 moles, de préférence de 1,1 à 1,8 mole par rapport au carbazole de formule IIa utilisé.

10. Procédé selon une ou plusieurs des revendications 1 à 5, caractérisé en ce qu'on fait réagir le N-éthoxycarbonylcarbazole de formule IIb 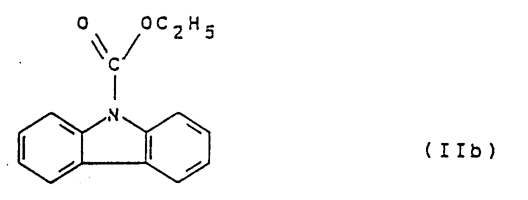 avec le carbonate de diéthyle en utilisant le carbonate de diéthyle dans une quantité de 0,1 à 1,0 mole, de préférence de 0,3 à 0,8 mole par rapport au carbazole de formule IIb utilisé et en mettant en oeuvre la réaction sans utilisation de solvant supplémentaire.
